# EUROPEAN PATENT SPECIFICATION

(11) **EP 3 991 703 B1**
(45) Date of publication and mention of the grant of the patent: **02.04.2025**
(21) Application number: 21204122.2
(22) Date of filing: 22.10.2021
(51) Int. Cl.: A61F 9/008

(54) **DEVICE CONFIGURED TO BE USED IN A LASIK SURGERY PROCEDURE**
VORRICHTUNG ZUR VERWENDUNG IN EINER LASIKOPERATION
DISPOSITIF CONÇU POUR ÊTRE UTILISÉ DANS UNE PROCÉDURE DE CHIRURGIE LASIK

(30) Priority: 27.10.2020 NL 2026779
(43) Date of publication of application: 04.05.2022
(73) Proprietor: Sharpsight B.V., 6049 MG Herten (NL)
(72) Inventor: Gonçalves, Arnaldo, 6049 MG Herten (NL)
(74) Representative: Dekker-Garms, Alwine Emilie

(56) References cited:
- RU-C1- 2 631 108
- US-A1- 2003 045 894
- US-B1- 6 220 246
- US-B1- 6 569 153

## Description

The invention relates to a device configured to be used in a LASIK surgery procedure.

LASIK surgery is a type of laser refractive surgery that is performed on the eye for the purpose of improving visual acuity and quality and to thereby remove the need for using external refractive accessories with the eye.

LASIK is the abbreviation of laser in situ keratomileusis and LASIK surgery is designed to correct refractive errors. LASIK surgery is commonly referred to as laser eye surgery or laser vision correction and is applied for the correction of myopia, hyperopia, and astigmatism. For most people, LASIK surgery provides a long-lasting alternative to glasses or contact lenses.

The LASIK surgery procedure involves steps of i) creating a corneal flap using a microkeratome or femtosecond laser, ii) reshaping the cornea using an excimer laser to remove tissue from the underlying stromal bed, and iii) putting the flap back in place.

As of 2018, roughly 9.5 million Americans have had LASIK surgery. Globally, between 1991 and 2016, more than 40 million LASIK surgeries were performed. However, LASIK surgery seems to be a declining option in the United States, and the number of surgeries has dropped more than 50%, from about 1.5 million surgeries in 2007 to 604,000 in 2015, according to the Market Scope eye-care data source.
A study in the *Cornea* journal determined the frequency with which LASIK was searched on Google from 2007 to 2011. Within this time frame, LASIK searched declined by 40% in the United States. Countries such as the UK and India also showed a decline, 22% and 24%, respectively. Canada, however, showed an increase in LASIK searches by 8%. By 2015 in the United States, LASIK declined by 50%. This decrease in interest can be attributed to several factors such as the emergence of refractive cataract surgery, the economic recession in 2008, and unfavorable media coverage from the FDA's 2008 press release on LASIK.

### Surgical Technique

Before LASIK surgery, the excimer laser, suction ring, microkeratome and blade (or femtosecond laser settings) are checked by the technician and the surgeon. The surgeon also confirms that the correct treatment data are entered into the laser computer. An eyelid speculum is placed in the operative eye, which has been anesthetized with drops, and the fellow eye is covered. The cornea is marked to aid in postoperative flap alignment. A suction ring is placed on the eye to achieve fixation. The microkeratome (or femtosecond laser) is used to create a hinged corneal flap. After the flap has been created, it is reflected away from the cut surface to an opened position. Excimer laser ablation is performed, centered on the pupil or on the corneal vertex. Eye-tracker and iris registration technology are increasingly used to ensure a well-centered laser treatment. Following the excimer laser ablation, the flap is put back in place.

### LASIK Complications

Complications occur in LASIK surgery as in any other surgical procedure. Serious adverse complications leading to significant permanent visual loss rarely occur. Less serious side effects such as dry eyes, night time starbursts, and/or reduced sensitivity occur relatively frequently.

The most common complication or side effect following LASIK surgery is dry eyes. Complications involving the LASIK flap include free, incomplete, or buttonholed flaps, striae/folds or slipped/displaced flaps. If the flap created during the LASIK surgery procedure is irregular, incomplete, or buttonholed, laser treatment cannot safely be performed in the same session. However, after a healing period, a secondary LASIK or PRK (photo-refractive keratomileusis) procedure may be performed in some cases. Flap-related complications are extremely rare when the flap is prepared using a femtosecond laser.

Complications that occur at the level of the interface between the flap and the stromal bed include diffuse lamellar keratitis, infection, and epithelial ingrowth. Post-LASIK ectasia may occur when the biomechanical stability of the cornea is altered. It is uncertain if there is any relationship between LASIK and an increased incidence of postoperative retinal detachment. Ischemic optic neuropathy is a rare complication that has been reported following LASIK.

### Outcomes of LASIK for Myopia and Astigmatism

For the correction of low to moderate myopia of less than -6D and low to moderate astigmatism of less than 2D, results from studies in the literature have shown that LASIK is effective and predictable in terms of obtaining very good to excellent uncorrected visual acuity, and that it is safe in terms of minimal loss of visual acuity. In a study evaluating the outcome of LASIK in high myopia, Reinstein et al showed that postoperative spherical equivalent was ±0.50 D in 55% and ±1.00 D in 83% of eyes after primary treatment. After retreatment, 69% of eyes where within ±0.50 D and 95% were within ±1.00 D.

Eyes with decentered ablations had a significantly higher magnitude of induced aberrations and lower uncorrected visual acuity than eyes with well-centered ablations. To minimize higher-order optical errors, special efforts to center the ablation zone are necessary, for example, by eye-tracking systems that consider the visual axis. Active eye tracking helps improve the visual outcome and reduces postoperative cylindrical astigmatism. With eye tracking, lasers used during LASIK surgery can compensate for eye movements during the procedure, adding an important level of assurance.

Postoperative residual or induced astigmatism may limit incorrected visual acuity (UCVA) and cause starbursts and glare. Irregular astigmatism can also cause loss of best-corrected visual acuity (BCVA), monocular diplopia, and ghosting of images. It has been suggested that even subtle irregularities in the corneal flap can reduce visual acuity postoperatively and even regular normal flaps may induce optical aberrations. Creating and handling the corneal flap are crucial steps in the LASIK surgery procedure. The hinge of the flap should be created at a slightly eccentric position by placing the suction ring at a slightly eccentric position, both in superior and nasal hinges, in order to avoid ablation of the flap during the procedure, especially in the case of a large ablation zone.

Following flap lifting and before ablation ensues, attention may be given to the "protection" of the flap during the procedure, in order to protect the flap from laser ablation. Although protecting the flap during laser ablation is an accepted practice in some clinics, not all surgeons routinely protect the flap during LASIK surgery, and this procedure has yet to be validated.

As mentioned, the purpose of flap protection is to avoid inadvertent flap ablation. Proper flap protection involves making sure that the whole inner surface of the flap that is in the opened position is shielded. Inadvertent flap ablation can cause ablation of both the stromal bed and the overlying stromal side of the flap leading to a double ablation effect. As described by Reinstein et al, this double ablation may cause over-flattening of the cornea in the hinge side and therefore cause irregular astigmatism.

It is generally known that protecting the corneal flap during myopic astigmatic LASIK may prevent double ablation and lead to better refractive outcomes. Currently, most surgeons use a sponge to shield the flap from the laser during ablation. This is associated with a better efficacy index and index of success when compared with the no-protection group. However, stabilization of movements is not obtained in this way, and protection of the flap is often incomplete and inadequate.

It is known that decentered ablations have as results various symptoms such as halos, ghost images, or night driving difficulties. Similar symptoms might also be caused by accidental flap ablation.

US6569153B1 discloses a multi-function surgical instrument for facilitating ophthalmic surgery of the eye by laser means. Included are a lower ring and an upper ring. The lower ring includes a central aperture to capture the limbus and aid in positioning of the eye. The upper ring is disposed above the surgical bed.

It is an objective of the invention to provide better eye centralization and stabilization by avoiding unexpected eye movement and better flap protection avoiding undesirable effects of a double ablation. In view thereof, the invention provides a device as defined in claim 1, which is a device that is capable of both ensuring eye fixation and protection of the corneal flap. The device has an eye fixating portion that is based on one, two, three or four points of scleral support around the cornea, and a flap protecting portion for completely covering the corneal flap. The various aspects of the invention will be apparent from and elucidated with reference to the following detailed description of a practical embodiment of a device comprising the eye fixating portion and the flap protecting portion as mentioned.

The invention will now be explained in greater detail with reference to the figures, in which equal or similar parts are indicated by the same reference signs, and in which:
Figures 1 and 2 diagrammatically show perspective views of a portion of a device according to the invention, and
Figures 3 and 4 illustrate an operational position of the device on an operative eye.

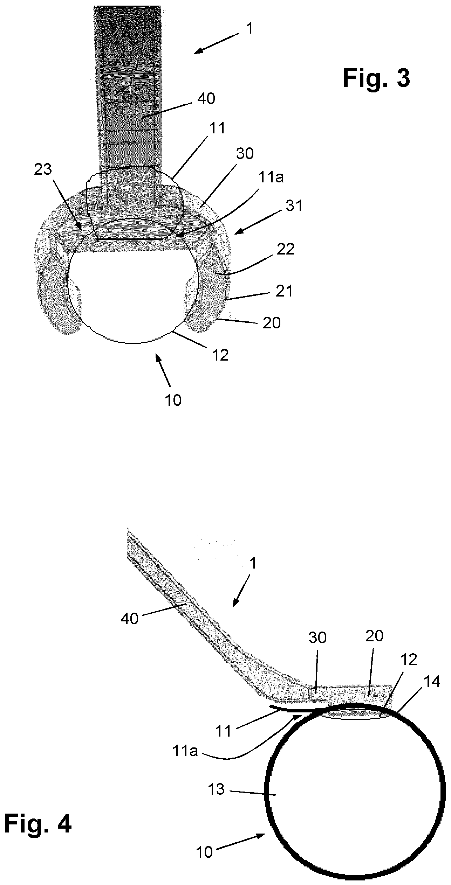

The device 1 according to the invention is configured to be used in a LASIK surgery procedure and to be put in an operational position on an operative eye 10 after a corneal flap 11 has been created and has been put to an opened position. As indicated in the foregoing, the operational position of the device 1 on the operative eye 10 is illustrated in figures 3 and 4. To this end, the cornea 12 and the corneal flap 11 of the eye 10 are diagrammatically depicted in figure 3, and the eyeball 13 and the corneal flap 11 of the eye 10 are diagrammatically depicted in figure 4.

The device 1 comprises an eye fixating portion 20 configured to engage on the sclera 14 around the cornea 12 and to thereby perform a fixating action on the eye 10 while enabling free access to the cornea 12 when the device 1 is in the operational position on the eye 10, and also comprises a generally plate-like flap protecting portion 30 configured to completely cover the corneal flap 11 in the opened position thereof when the device 1 is in the operational position on the eye 10. Further, the device 1 comprises a handle portion 40. In this respect, it is noted that only a part of the handle portion 40 is shown in the figures. In the shown example, the handle portion 40 is connected to the flap protecting portion 30 and has a curved appearance at the position of connection to the flap protecting portion 30.

The eye fixating portion 20 comprises at least two ridge-like support elements 21 having a contact surface 22 through which the eye fixating portion 20 is to contact the sclera 14 when the device 1 is in the operational position on the eye 10. In the framework of the invention, the number of support elements 21 may be chosen freely, wherein two, three or four are practical examples. In the shown embodiment of the device 1 according to the invention, the eye fixating portion 20 comprises two curved support elements 21 configured to contact the sclera 14 at opposite sides of the cornea 12.

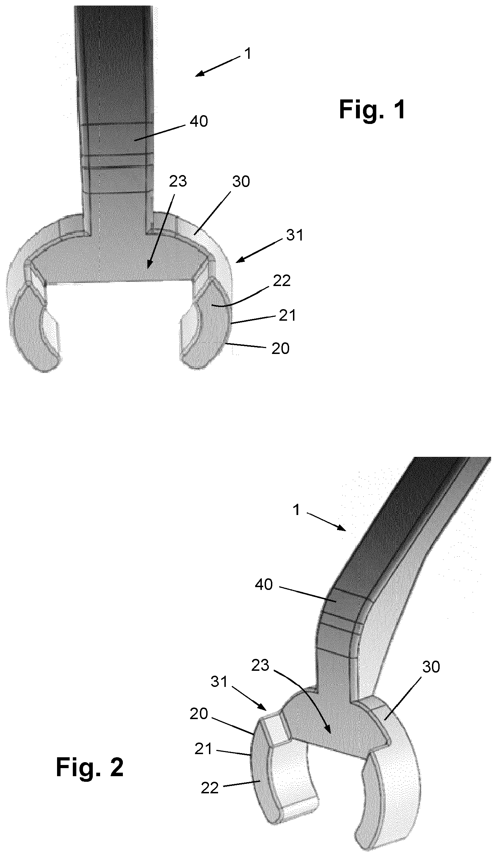

In figure 3, it can be seen that a space 23 between the two support elements 21 along the flap protecting portion 30 is configured to accommodate a hinge portion 11a of the corneal flap 11. In figures 1, 2 and 3, it can be seen that at a side of the device 1 that is configured to face the eye 10, the contact surface 22 of the respective support elements 21 is at a protruding level relative to the flap protecting portion 30.

In the embodiment of the device 1 according to the invention shown in the figures, the flap protecting portion 30 is generally shaped as a minor segment of a circle. As can be seen in figures 1, 2 and 3, the two support elements 21 extend from opposite corner areas 31 of the flap protecting portion 30 in this embodiment.

Advantageously, the eye fixating portion 20 and the flap protecting portion 30 are made as a single integral entirety. It is also possible that the eye fixating portion 20, the flap protecting portion 30 and the handle portion 40 are made as a single integral entirety, which implies that the device 1 may be provided as a single integral entirety. On the other hand, an option such as the handle portion 40 being releasably coupled to the flap protecting portion 30 is also feasible in the framework of the invention.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims.

While the invention has been illustrated and described in detail in the figures and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

## Claims

1. Device (1) configured to be used in a LASIK surgery procedure and to be put in an operational position on an operative eye (10) after a corneal flap (11) has been created and has been put to an opened position, comprising:
- an eye fixating portion (20) configured to engage on the sclera (14) around the cornea (12) and to thereby perform a fixating action on the eye (10) while enabling free access to the cornea (12) when the device (1) is in the operational position on the eye (10), and
- a flap protecting portion (30) configured to completely cover the corneal flap (11) in the opened position thereof when the device (1) is in the operational position on the eye (10),
wherein the eye fixating portion (20) comprises at least two support elements (21) having a contact surface (22) through which the eye fixating portion (20) is to contact the sclera (14) when the device (1) is in the operational position on the eye (10),
**characterized in that** a space (23) between two support elements (21) along the flap protecting portion (30) is configured to accommodate a hinge portion (11a) of the corneal flap (11).

2. Device (1) according to claim 1, wherein the eye fixation portion (20) comprises two, three or four support elements (21).

3. Device (1) according to claim 1 or 2, wherein the eye fixating portion (20) comprises two curved support elements (21) configured to contact the sclera (14) at opposite sides of the cornea (12).

4. Device (1) according to any of claims 1-3, wherein, at a side of the device (1) that is configured to face the eye (10), the contact surface (22) of the at least one support element (21) is at a protruding level relative to the flap protecting portion (30).

5. Device (1) according to any of claims 1-4, wherein the flap protecting portion (30) is generally shaped as a minor segment of a circle.

6. Device (1) according to claim 5, wherein two support elements (21) extend from opposite corner areas (31) of the flap protecting portion (30).

7. Device (1) according to any of claims 1-6, further comprising a handle portion (40).

8. Device (1) according to claim 7, wherein the handle portion (40) is connected to the flap protecting portion (30).

9. Device (1) according to claim 8, wherein the handle portion (40) has a curved appearance at the position where the handle portion (40) is connected to the flap protecting portion (30).

10. Device (1) according to any of claims 1-9, wherein the eye fixating portion (20) and the flap protecting portion (30) are made as a single integral entirety.

11. Device (1) according to claim 10 insofar as dependent on any of claims 7-9, wherein the eye fixating portion (20), the flap protecting portion (30) and the handle portion (40) are made as a single integral entirety.

## Patentansprüche

1. Vorrichtung (1), die so konfiguriert ist, dass sie in einem LASIK-Operationsverfahren verwendet und in eine Betriebsposition auf einem zu operierenden Auge (10) gebracht werden kann, nachdem ein Hornhautlappen (11) erzeugt und in eine geöffnete Position gebracht worden ist, umfassend:
- einen Augenfixierabschnitt (20), der so konfiguriert ist, dass er an der Sklera (14) um die Hornhaut (12) herum angreift und dadurch eine Fixierwirkung auf das Auge (10) ausübt, während er freien Zugang zur Hornhaut (12) ermöglicht, wenn sich die Vorrichtung (1) in der Betriebsposition auf dem Auge (10) befindet, und
- einen Lappenschutzabschnitt (30), der so konfiguriert ist, dass er den Hornhautlappen (11) in seiner geöffneten Position vollständig abdeckt, wenn sich die Vorrichtung (1) in der Betriebsposition auf dem Auge (10) befindet,
wobei der Augenfixierabschnitt (20) mindestens zwei Stützelemente (21) mit einer Kontaktfläche (22) umfasst, durch die der Augenfixierabschnitt (20) die Sklera (14) berühren soll, wenn sich die Vorrichtung (1) in der Betriebsposition auf dem Auge (10) befindet, **dadurch gekennzeichnet, dass** ein Raum (23) zwischen zwei Stützelementen (21) entlang des Lappenschutzabschnitts (30) so konfiguriert ist, dass er einen Gelenkabschnitt (11a) des Hornhautlappens (11) aufnimmt.

2. Vorrichtung (1) nach Anspruch 1, wobei der Augenfixierungsabschnitt (20) zwei, drei oder vier Stützelemente (21) umfasst.

3. Vorrichtung (1) nach Anspruch 1 oder 2, wobei der Augenfixierabschnitt (20) zwei gekrümmte Stützelemente (21) umfasst, die so konfiguriert sind, dass sie die Sklera (14) auf gegenüberliegenden Seiten der Hornhaut (12) berühren.

4. Vorrichtung (1) nach einem der Ansprüche 1 bis 3, wobei an einer Seite der Vorrichtung (1), die so gestaltet ist, dass sie dem Auge (10) zugewandt ist, die Kontaktfläche (22) des mindestens einen Stützelements (21) in Bezug auf den Lappenschutzabschnitt (30) vorstehend ist.

5. Vorrichtung (1) nach einem der Ansprüche 1 bis 4, wobei der Lappenschutzabschnitt (30) im Allgemeinen die Form eines kleinen Kreissegments aufweist.

6. Vorrichtung (1) nach Anspruch 5, wobei sich zwei Stützelemente (21) von gegenüberliegenden Eckbereichen (31) des Lappenschutzabschnitts (30) erstrecken.

7. Vorrichtung (1) nach einem der Ansprüche 1 bis 6, ferner umfassend einen Griffteil (40).

8. Vorrichtung (1) nach Anspruch 7, wobei der Griffteil (40) mit dem Lappenschutzabschnitt (30) verbunden ist.

9. Vorrichtung (1) nach Anspruch 8, wobei der Griffteil (40) an der Stelle, an der der Griffteil (40) mit dem Lappenschutzabschnitt (30) verbunden ist, ein gekrümmtes Aussehen aufweist.

10. Vorrichtung (1) nach einem der Ansprüche 1 bis 9, wobei der Augenfixierabschnitt (20) und der Lappenschutzabschnitt (30) als ein einziges integrales Ganzes hergestellt sind.

11. Vorrichtung (1) nach Anspruch 10, insofern sie von einem der Ansprüche 7 bis 9 abhängt, wobei der Augenfixierabschnitt (20), der Lappenschutzabschnitt (30) und der Griffteil (40) als ein einziges integrales Ganzes hergestellt sind.

## Revendications

1. Dispositif (1) configuré pour être utilisé dans une procédure de chirurgie LASIK et pour être mis dans une position opérationnelle sur un œil fonctionnel (10) après qu'un capot cornéen (11) a été créé et a été mis dans une position ouverte, comprenant:
- une partie de fixation d'œil (20) configurée pour entrer en contact avec la sclère (14) autour de la cornée (12) et pour réaliser ainsi une action de fixation sur l'œil (10) tout en permettant un accès libre à la cornée (12) lorsque le dispositif (1) est dans la position opérationnelle sur l'œil (10), et
- une partie de protection de capot (30) configurée pour couvrir complètement le capot cornéen (11) dans la position ouverte de ce dernier lorsque le dispositif (1) est dans la position opérationnelle sur l'œil (10),
dans lequel la partie de fixation d'œil (20) comprend au moins deux éléments de support (21) ayant une surface de contact (22) par l'intermédiaire de laquelle la partie de fixation d'œil (20) doit entrer en contact avec la sclère (14) lorsque le dispositif (1) est dans la position opérationnelle sur l'œil (10), **caractérisé en ce qu'**un espace (23) entre deux éléments de support (21) le long de la partie de protection de capot (30) est configuré pour recevoir une partie charnière (11a) du capot cornéen (11).

2. Dispositif (1) selon la revendication 1, dans lequel la partie de fixation d'œil (20) comprend deux, trois ou quatre éléments de support (21).

3. Dispositif (1) selon la revendication 1 ou 2, dans lequel la partie de fixation d'œil (20) comprend deux éléments de support courbés (21) configurés pour entrer en contact avec la sclère (14) sur des côtés opposés de la cornée (12).

4. Dispositif (1) selon l'une quelconque des revendications 1 à 3, dans lequel, sur un côté du dispositif (1) qui est configuré pour faire face à l'œil (10), la surface de contact (22) dudit au moins un élément de support (21) est à un niveau saillant par rapport à la partie de protection de capot (30).

5. Dispositif (1) selon l'une quelconque des revendications 1 à 4, dans lequel la partie de protection de capot (30) a la forme globale d'un petit segment d'un cercle.

6. Dispositif (1) selon la revendication 5, dans lequel deux éléments de support (21) s'étendent depuis des régions de coins opposées (31) de la partie de protection de capot (30).

7. Dispositif (1) selon l'une quelconque des revendications 1 à 6, comprenant en outre une partie formant poignée (40).

8. Dispositif (1) selon la revendication 7, dans lequel la partie formant poignée (40) est reliée à la partie de protection de capot (30).

9. Dispositif (1) selon la revendication 8, dans lequel la partie formant poignée (40) a une apparence courbée à la position où la partie formant poignée (40) est reliée à la partie de protection de capot (30).

10. Dispositif (1) selon l'une quelconque des revendications 1 à 9, dans lequel la partie de fixation d'œil (20) et la partie de protection de capot (30) sont faites d'un seul tenant.

11. Dispositif (1) selon la revendication 10 lorsqu'elle dépend de l'une quelconque des revendications 7 à 9, dans lequel la partie de fixation d'œil (20), la partie de protection de capot (30) et la partie formant poignée (40) sont faites d'un seul tenant.
